Europäisches Patentamt

European Patent Office

Office européen des brevets

(11) Publication number: **0 232 074 B1**

# EUROPEAN PATENT SPECIFICATION

(45) Date of publication of patent specification: **17.04.91**      (51) Int. Cl.⁵: **A61M 25/01**

(21) Application number: **87300532.6**

(22) Date of filing: **22.01.87**

(54) **Medical tube.**

(30) Priority: **30.01.86 US 823918**

(43) Date of publication of application:
**12.08.87 Bulletin 87/33**

(45) Publication of the grant of the patent:
**17.04.91 Bulletin 91/16**

(84) Designated Contracting States:
**AT BE CH DE ES FR GB IT LI LU NL SE**

(56) References cited:
**EP-A- 0 159 773**
**DE-A- 2 936 655**
**US-A- 2 458 305**
**US-A- 3 154 080**
**US-A- 4 316 459**

(73) Proprietor: **SHERWOOD MEDICAL COMPANY**
**1831 Olive Street**
**St. Louis, MO 63103(US)**

(72) Inventor: **Fecht, David C.**
**1025 Brittany Parkway**
**Manchester Missouri 63011(US)**

(74) Representative: **Porter, Graham Ronald et al**
**c/o Wyeth Laboratories Huntercombe Lane**
**South**
**Taplow Maidenhead Berkshire SL6 0PH(GB)**

Rank Xerox (UK) Business Services

## Description

This invention relates to a medical tube and more particularly to a tip for an aortic cannula.

Aortic cannulas are used during cardiac surgery to convey blood from an extracorporeal circulation system to the aorta. An aortic cannula can; for example; be employed in a total bypass so that the heart or other organs and vessels can be operated on in a substantially dry state.

An aortic cannula generally comprises a flexible tube having a relatively rigid tip which is forced through an incision in the wall of the aorta. It is important that the incision is no larger than absolutely necessary to minimise leakage and subsequent healing time.

Other medical tubes, for example for insertion into other blood vessels, have similar requirements. EPA-159773 describes a catheter comprising an elongate tube having a tip attached to the distal end thereof wherein the tip comprises a proximal portion attached to the tube and having a plurality of longitudinally extending struts connected to the base of a generally conical member whose apex is towards said tube.

One problem with conventional aortic cannulas is that the tip is rather difficult to insert through the aortic incision without causing damage to the aorta. Generally the tip is rounded and blunt to avoid cutting the aorta but the blunt tip may nevertheless traumatize the aorta wall as it is pushed through the incision. If the incision is made large enough to reduce trauma during insertion of the cannula tip, then there is of course, greater damage to the patient due to the enlarged incision, and such an incision requires a greater number of stitches and may have a longer healing time. The user has the choice of either risking considerable blood leakage by making a large incision or damaging the aorta due to the forces required to insert the cannula.

In some cases, open-ended cannulas are employed; with such cannulas the edge of the wall about the end opening tends to catch on the edge of the incision and damage the aorta. Some open-ended aortic cannulas have angled ends for effecting a change in the direction of blood flow from the cannula to the aorta. These angled end devices, however, must be manipulated for proper orientation during insertion and may cause further damage to the aorta.

Similar problems are encountered in the insertion of medical tubes into other vessels.

It is therefore an object of the present invention to provide a medical tube having an improved distal end adapted for insertion through an incision in a patient and wherein one or more of the above-mentioned problems or disadvantages are overcome.

According to the invention there is provided a tip for a medical tube and comprising a tubular member having proximal and distal openings for the passage of fluid therethrough, the tip having a longitudinally extending ridge on the outer periphery thereof, wherein said ridge is the sole longitudinally extending ridge on the tip and blends smoothly into the distal end of said tip.

The ridge ensures that the surgical incision into which the tip is placed is gradually dilated; this ensures minimum trauma to the patient since force required to insert the tip is reduced, and minimum blood loss since the incision is gradually stretched around the tip to give an improved seal. The tip allows the surgeon to make the incision smaller than hitherto whilst achieving a minimum level of patient trauma and blood loss.

The ridge may be defined by opposite recesses in the tip wall which are preferably eliptical in shape. All exterior surfaces of the tip blend smoothly into one another to ensure ease of insertion and minimise the chance of cutting or tearing the area of the incision.

In the preferred embodiment the distal opening of the tip is on the opposite side to the ridge. The tip tapers radially inwardly at the distal end to a smoothly rounded blunt point. The tip lumen preferably blends smoothly from proximal to distal openings to ensure smooth blood flow and reduce hemolysis.

A peripheral flange may be provided on the tip to limit insertion through the incision; the flange may be secured to the area of the incision, by for example, sutures through holes herein.

The invention also provides a medical tube comprising a tube and the tip aforesaid. The tube is preferably connected to the proximal end of the tip and abuts a circular internal abutment so that the bore of the tube matches the bore of the tip lumen to ensure smooth blood flow therebetween.

The invention includes an aortic cannula including the tip described herein and other features described below.

Other features of the invention will be apparent from the following description of the preferred embodiment shown, by way of example only, the accompanying drawings in which:-

Figure 1 is a diagrammatic illustration of a human heart and some associated blood vessels connected to an extracorporeal circulation system and including an aortic cannula having a tip according to the present invention;

Figure 2 is an elevation of the aortic cannula of Figure 1 with a proximal connector attached thereto;

Figure 3 is an enlarged elevation of the tip of the cannula shown in Figure 2;

Figure 4 is a top view of the cannula tip shown in Figure 3;
Figure 5 is a view of the underside of the cannula tip shown in Figure 3;
Figure 6 is a right end view of the tip as viewed in Figure 3;
Figure 7 is a cross-section along line 7-7 of Figure 5 and showing the tip attached to the cannula;
Figure 8 is a cross-section along line 8-8 of Figure 3;
Figure 9 is an enlarged plan view of a portion of the aorta shown in Figure 1 showing a suitable surgical incision;
Figures 10 and 11 illustrate one manner of inserting the tip into the incision of Figure 9.

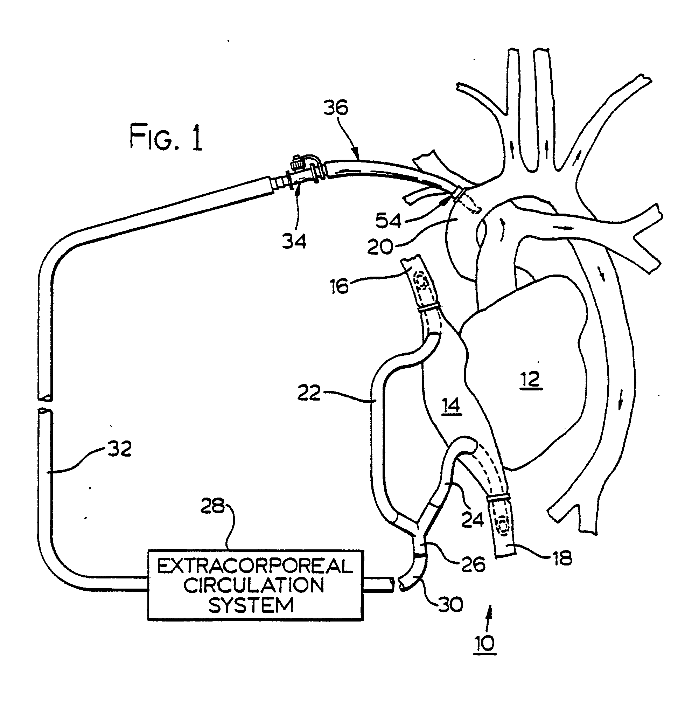

Referring now to the drawings, and particularly to Figure 1, there is illustrated a portion of a surgical site 10 showing a heart 12, a right atrium 14, superior and inferior vena cavae 16 and 18 respectively, and an aorta 20 of a patient. A pair of vena caval catheters 22 and 24 extend into the atrium 14 and into the vena cavae 16 and 18, respectively. The vena cavae may be tightened about the ends of the catheters 22 and 24 by suitable noose or the like as shown.

The opposite ends of the vena caval catheters 22 and 24 are connected through a Y-connector 26 and tube 30 to the inlet of an extracorporeal circulation system 28. The extracorporeal system may include a blood oxygenator, a blood pump, filters, bubble removing apparatus, and a defoamer. System 28 serves as an artificial heart and lung, changing venous blood into oxygenated blood. A tube 32 connects the outlet side of the circulation system 28 via a tube connector 34 to an aortic cannula 36, shown inserted into the aorta 20, for returning oxygenated blood to the arterial system of the patient. The extracorporeal circulation system in Figure 1, completely bypasses the heart so that the heart or associated organs and vessels may be operated on in a substantially dry state.

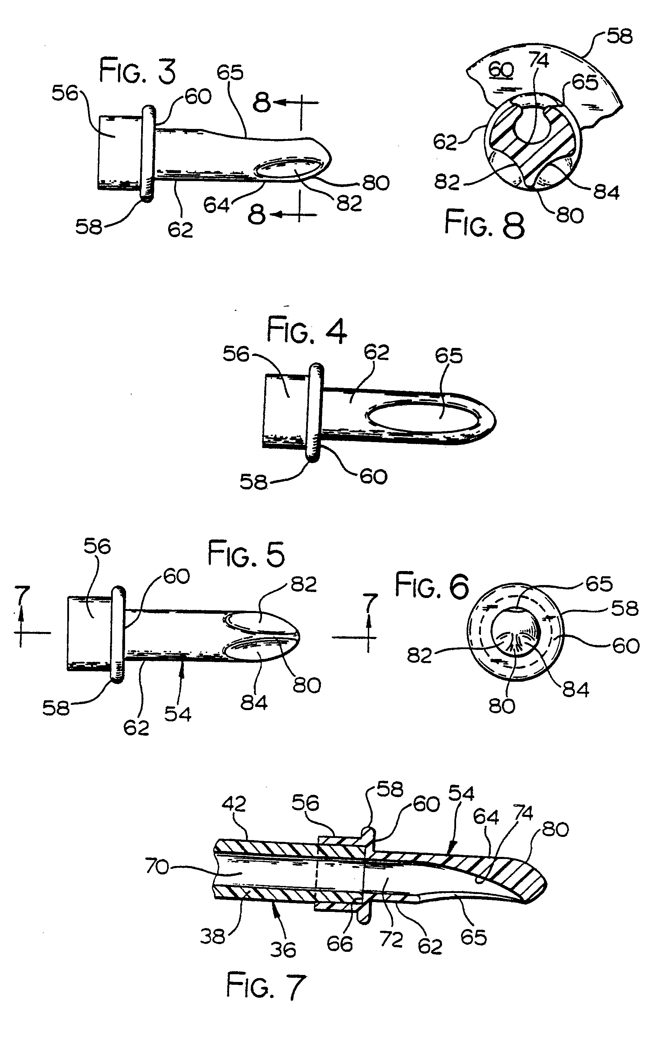

The aortic cannula 36, as seen in Figure 2, includes a tube 38 tapering slightly from the proximal or left end 40 radially inwardly in the distal direction. A hollow tube connector 34 has one end inserted into the proximal end 40 in tight sealing engagement. A removable end cap 46 is shown in sealing engagement in the proximal end of connector 34. The cap 46 is shown having a passage 47 extending therethrough; a hydrophobic filter 48 covers the distal end of passage 47 while the proximal end of the passage is open to the atmosphere. The cap 46, as will be further described, allows air in the cannula 36 to be purged during insertion procedures but will not allow blood to pass. The connector 34 is provided with a side port 49 that is shown closed by a cap 50 which is tethered by a resilient strap 51 having an eyelet 52 surrounding the connector 34. The port 49 may be provided with a female luer taper for connection to other apparatus, for example, for blood sampling. The port 49 may be provided with conventional luer lock ears and the inner wall of cap 50 provided with complementary luer lock threads so tha t cap 50 can be threaded onto and off of connector 34 as desired.

Cannula 36 includes a tip 54 connected to the distal end 42 of tube 38. Preferably, tip 54 is formed or moulded as a separate plastic element and attached to the tube 38. The tube 38 may be made of any suitable plastic or rubber; preferably one that is flexible enough to allow some bending but which does not easily kink and occlude the fluid passage when moderate bending forces are applied to it, such as during the connection of the cannula in the circulation system. Tube 38 may be formed of, for example, polyvinyl chloride. The tip 54 may also be formed of any suitable material, for example, the same material as tube 38 but preferably of a somewhat harder or more rigid grade so that it can be inserted into the aorta without bending. Tip 54 may be moulded, for example, from a relatively rigid polyvinyl chloride.

Referring especially to Figures 3 to 8, tip 54 comprises an annular collar 56 having a circular flange 58 that has a distally facing flat side 60 and may be provided with suture slots if desired for securing the tip to the aorta. Collar 56 is integrally connected with a distally extending, generally cylindrical portion 62 of the tip which smoothly connects with a distal end portion 64. An elongate or generally eliptical opening 65 (Figure 4) is provided through the wall of the tip. As best seen in Figure 7, the distal end portion 42 of tube 38 is shown extending into collar 56 and engaging an inner radially inwardly extending circular wall or land 66. The wall 66 and the outer wall of the tube 38 may be fixed together, such as by an adhesive, solvent bonding, or by other suitable means. Preferably, and as shown for illustration in Figure 7, the thickness of the sidewall of tube 38 at the distal end is substantially the same as the width of the circular wall 66 so that the tube lumen, indicated at 70, and the lumen of the cylindrical portion 62 of the tip, indicated at 72, are substantially the same diameter so as to provide a smooth transition for blood flow from the tube 38 to the tip 54.

The distal end portion 64 has an inner preferably smoothly curving wall 74 (Figure 7) extending between the inner wall of the cylindrical portion 62 and the distal end of opening 65 so that blood flowing distally in lumen 70 flows into the tip 54 and out the opening 65 with minimal turbulence even though there is a substantial angular change in the direction of blood flow. The wall 74 closes the distal end of the tip 54 and directs the flow of blood out of opening 65.

The distal end portion 64 is provided with a smoothly contoured ridge 80 in the outer surface of the tip formed by two smoothly curving generally eliptical cavities or recesses 82 and 84 in the outer surface of the tip on opposite sides and adjacent the ridge 80; the recesses are symmetrical. Ridge 80 smoothly blends into the cylindrical portion 62 of the tip as well as the distal extremity of the tip. The ridge 80 and recesses 82 and 84 have smoothly curving edges as best seen in Figure 8 and the outer surface of the tip is consequently free of any sharp edge. The edge of the opening 65 may be radiused or rounded to smoothly blend with the outer surface of the tip. The major axis of the recesses 82 and 84 extends longitudinally of the tip and curves radially inwardly toward the distal end. In this way, the exterior surfaces of the tip adjacent each side of the ridge 80 taper radially inwardly toward the distal end. The radially outermost surface of the ridge 80, as best seen in Figure 3 and 7, is coextensive with the outer surface of the cylindrical portion 62 of the tip.

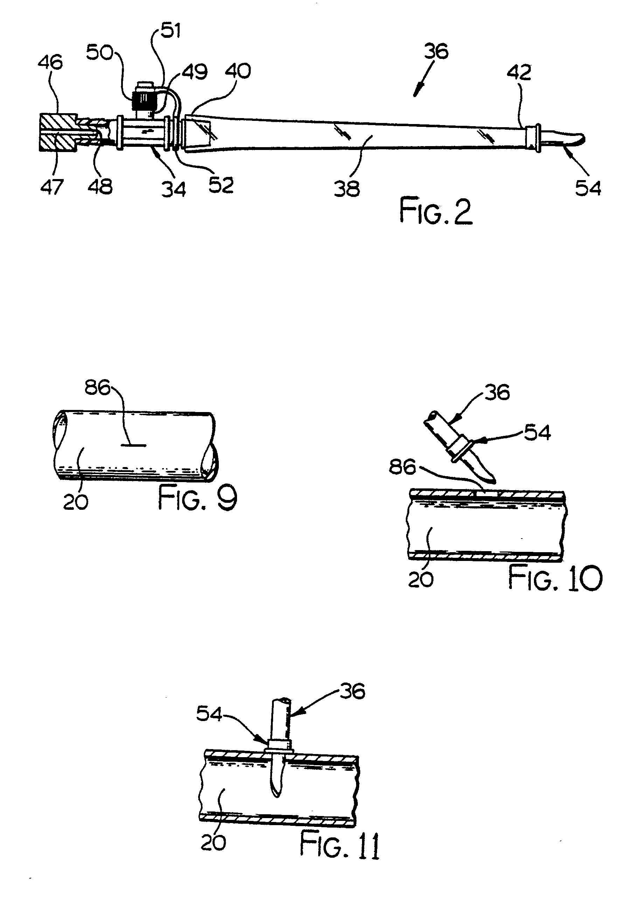

Preparatory to insertion, an incision or a slit 86, as shown in Figure 9, may be made in the aorta 20. In use the cannula is held with ridge 80 at the bottom of the tip as shown in Figure 10; the ridge is moved toward the slit with the cannula being held at an angle to the longitudinal axis of the aorta. As the ridge enter the slit, the slit is opened gradually or dilated until the entire tip penetrates the wall of the aorta. The cannula is then moved into the aorta until the annular distal side 60 of the flange 58 engages the outer surface of the aorta as shown in Figure 11. The flange 58 may be sutured to the aorta.

Upon insertion of the tip into the aorta 20, blood from the aorta flows into the cannula 36 displacing the air in the cannula 36 and causing the air to flow through the filter passage 47 to the atmosphere. The hydrophobic filter 48 will not allow blood to pass. With the air removed from the cannula, cap 46 is removed and the proximal end of connector 34 connected to the tube 32 (Figure 1). The tube 38 may conveniently be clamped off during removal of the cap and the connection of tube 32 to tube 38. If further air is found in the cannula 36 or connector 34, it may be vented from port 49.

By providing the longitudinally extending smoothly blending ridge 80, the ridge can be used as the leading edge of the cannula during insertion into the aorta so that the forces applied are more evenly distributed in spreading the walls of the incision thereby reducing trauma to the aorta and reducing blood loss. Upon insertion of the tip 54, the slit will tend to be dilated and conform closely to the outer wall of the cylindrical portion 62. The tip 38 not only reduces blood loss during insertion of the cannula but also reduces blood loss as the operation continues. By providing a smoothly curving lumen and eliminating sharp edges, blood can flow through the cannula with reduced hemolysis.

As various changes could be made in the above construction without departing from the scope of the invention, it is intended that all matter contained in the above description and apparatus shown in the accompanying drawings shall be interpreted as illustrative and not in a limiting sense.

## Claims

1. A tip (54) for a medical tube and comprising a tubular member having proximal and distal openings for the passage of fluid therethrough, the tip (54) having a longitudinally extending ridge on the outer periphery thereof which blends smoothly into the distal end of said tip (54), characterised in that the ridge (80) is the sole longitudinally extending ridge on the tip.

2. A tip according to Claim 1, wherein said ridge (80) is defined by opposite recesses (82, 84) in the tip wall.

3. A tip according to Claim 2, wherein said recesses (82, 84) are substantially eliptical.

4. A tip according to Claim 3, wherein the major axis of said recesses (82, 84) tapers toward the geometric centre of the tip in a distal direction.

5. A tip according to any preceding claim, wherein said distal opening (65) is in the sidewall of the tip and is the sole opening therein.

6. A tip according to any preceding claim, wherein said distal opening (65) is on the opposite side of the tip to said ridge (80).

7. A tip according to Claim 6, wherein the tip has a lumen (72) which curves smoothly from the proximal opening to the distal end of the distal opening.

8. A tip according to Claim 7, and including a peripheral flange (58) to limit insertion of the tip into a body incision.

9. A tip according to Claim 8, wherein said flange (58) is intermediate the ends thereof.

**10.** A medical tube comprising a tube (38) having a tip (54) according to any preceding claim, wherein the tube is connected to the proximal end of the tip.

**11.** A medical tube according to Claim 10, wherein the tip has a lumen (72) which blends smoothly into a lumen (70) in the tube (38).

**12.** An aortic cannula (36) for insertion into an incision made in an aorta of a patient for connecting the aorta into an extra corporeal blood circulation system comprising a plastic tube (38) having a lumen (70) extending therethrough and proximal and distal ends, means for connecting the proximal end (40) of said tube (38) into the extra corporeal blood ciculation system, and a generally tubular plastic tip (54) having a proximal end connected to the distal end of said tube, said tip (54) having a lumen (72) connected with said tube lumen (70) and an opening through the side wall thereof for the flow of blood therethrough, said tip lumen (72) having a distal end wall closing the distal end thereof, a ridge (80) having smoothly rounded generally extending edges smoothly blending into the outer surface of the distal end of said tip (54), the tip (54) being of a relatively hard material such that the material maintains said tip (54) in a fixed shape during use characterised in that the distal end wall smoothly curves from the sidewall of said tip lumen (72) to the distal end of said opening, said ridge (80) is the only ridge on said tip, said tip having a pair of elongate recesses (82, 84) in the outer surface thereof adjacent the distal end thereof defining the generally external axially extending ridge (80) between said recesses (82,84) for moving the walls of the aorta apart at the incision, said opening being the only opening in the side wall of said tip, said ridge (80) being located on the side of said tip opposite the side that includes said opening said tip having a generally cylindrical portion (62) proximally of the proximal end of said opening, the radially outermost surfaces of said cylindrical portion (62) and of said ridge (80) being substantially radially equidistant from the longitudinal axis of said tip (54), the distal end of said tip (54) having an outer smoothly curving surface between the distal end of said opening and the distal end of said ridge (80), and a flange (58) connected to said cylindrical portion (62) extending radially outwardly therefrom and located a predetermined distance from the distal end surface of said tip (54) to limit the distance of insertion of said tip (54) into the aorta wherein said recesses are inwardly concave.

**13.** A cannula (36) as claimed in Claim 12, wherein said recesses (82, 84) are substantially eliptical with their longer axes extending generally inwardly and toward the distal end of said tip (54).

**14.** A cannula (36) as claimed in Claim 13, wherein said recesses (82,84) are arcuate in cross-section.

**Revendications**

1. Pointe (54) pour un tube médical et comprenant un organe tubulaire comportant des ouvertures proximale et distale pour le passage d'un fluide à l'intérieur, la pointe (54) comprenant une nervure qui s'étend longitudinalement sur sa périphérie extérieure et qui se perd en contour lisse dans l'extrémité distale de la pointe (54), caractérisée en ce que la nervure (40) est l'unique nervure qui s'étend longitudinalement sur la pointe.

2. Pointe suivant la revendication 1, dans laquelle la nervure (80) est définie par des creux opposés (82, 84) dans la paroi de la pointe.

3. Pointe suivant la revendication 2, dans laquelle les creux (82, 84) sont sensiblement elliptiques.

4. Pointe suivant la revendication 3, dans laquelle l'axe principal des creux (82, 84) se rapproche du centre géométrique de la pointe en direction distale.

5. Pointe suivant l'une quelconque des revendications précédentes, dans laquelle l'ouverture distale (65) est pratiquée dans la paroi latérale de la pointe et est l'unique ouverture pratiquée.

6. Pointe suivant l'une quelconque des revendications précédentes, dans laquelle l'ouverture distale (65) est pratiquée dans la face de la pointe opposée à la nervure (80).

7. Pointe suivant la revendication 6, dans laquelle la pointe comporte une lumière (72) qui s'incurve en contour lisse de l'ouverture proximale jusqu'à l'extrémité distale de l'ouverture distale.

8. Pointe suivant la revendication 7, et comprenant une bride périphérique (58) pour limiter l'insertion de la pointe dans une incision du corps.

9. Pointe suivant la revendication 8, dans laquelle la bride (58) est intermédiaire entre ses extrémités.

10. Tube médical comprenant un tube (38) muni d'une pointe (54) suivant l'une quelconque des revendications précédentes, dans lequel le tube est connecté à l'extrémité proximale de la pointe.

11. Tube médical suivant la revendication 10, dans lequel la pointe comporte une lumière (72) qui se continue en contour lisse dans une lumière (70) du tube (38).

12. Canule aortique (36) pour l'insertion dans une incision pratiquée dans l'aorte d'un patient pour connecter l'aorte à un système de circulation extracorporelle du sang, comprenant un tube en matière plastique (38) comportant une lumière (70) qui s'étend dans celui-ci et des extrémités proximale et distale, des moyens pour connecter l'extrémité proximale (40) du tube (38) au système de circulation extracorporelle du sang et une pointe généralement tubulaire en matière plastique (54) comprenant une extrémité proximale connectée à l'extrémité distale du tube, la pointe (54) comportant une lumière (72) communiquant avec la lumière (70) du tube et une ouverture pratiquée dans sa paroi latérale pour le passage du sang dans celle-ci, la lumière (72) de la pointe comprenant une paroi d'extrémité distale fermant son extrémité distale, une nervure (80) présentant des bords qui s'étendent de façon générale en contour lisse et se perdent en contour lisse dans la surface extérieure de l'extrémité distale de la pointe (54), la pointe (54) étant faite d'une matière relativement dure de façon que la matière maintienne la pointe (54) à une forme fixe en service, caractérisée en ce que la paroi d'extrémité distale s'incurve en contour lisse depuis la paroi latérale de la lumière (72) du tube jusqu'à l'extrémité distale de l'ouverture, la nervure (80) est l'unique nervure sur la pointe, la pointe comprenant deux creux oblongs (82, 84) ménagés dans sa paroi extérieure en position adjacente à son extrémité distale et définissant la nervure externe (80) qui s'étend de façon générale axialement entre les creux (82, 84) pour écarter les parois de l'aorte à l'incision, l'ouverture étant l'unique ouverture dans la paroi latérale de la pointe, la nervure (80) étant située du côté de la pointe opposé au côté qui comprend l'ouverture, la pointe comprenant une partie généralement cylindrique (62) en position proximale de l'extrémité proximale de l'ouverture, les surfaces radialement extérieures de la partie cylindrique (62) et de la nervure (80) étant en substance radialement équidistantes de l'axe longitudinal de la pointe (54), l'extrémité distale de la pointe (54) comprenant une surface extérieure en courbe lisse entre l'extrémité distale de l'ouverture et l'extrémité distale de la nervure (80), et une bride (58) qui est adaptée à la partie cylindrique (62) et qui s'étend radialement vers l'extérieur de celle-ci et est située à une distance déterminée au préalable de la surface d'extrémité distale de la pointe (54) pour limiter la profondeur d'insertion de la pointe (54) dans l'aorte, les creux étant concaves vers l'intérieur.

13. Canule (36) suivant la revendication 12, dans laquelle les creux (82, 84) sont sensiblement elliptiques avec leurs grands axes qui s'étendent de façon générale vers l'intérieur et vers l'extrémité distale de la pointe (54).

14. Canule (36) suivant la revendication 13, dans laquelle les creux (82, 84) ont une section transversale arquée.

**Ansprüche**

1. Spitzes Endstück (54) für eine medizinische Schlauchleitung mit einem schlauchförmigen Teil, das proximale und distale Öffnungen für den Durchfluß einer Flüssigkeit besitzt, wobei das spitze Endstück eine sich longitudinal erstreckende Rippe auf seiner äußeren Peripherie besitzt, die glatt in das distale Ende des spitzen Endstückes (54) übergeht,
**dadurch gekennzeichnet,**
daß die Rippe (80) die einzige sich longitudinal erstreckende Rippe auf dem spitzen Endstück ist.

2. Spitzes Endstück nach Anspruch 1, dadurch gekennzeichnet, daß die Rippe (80) durch gegenüberliegende Ausnehmungen (82, 84) in der Endstückwand definiert ist.

3. Spitzes Endstück nach Anspruch 2, gekennzeichnet durch im wesentlichen elliptische Ausnehmungen (82, 84).

4. Spitzes Endstück nach Anspruch 3, dadurch gekennzeichnet, daß die Hauptachse der Ausnehmungen (82, 84) sich zu dem geometrischen Zentrum des Endstückes in einer distalen Richtung verjüngt.

5. Spitzes Endstück nach einem der vorhergehenden Ansprüche, dadurch gekennzeichnet, daß die distale Öffnung (65) in der Seitenwand des spitzen Endstückes angeordnet und die einzige Öffnung hierin ist.

6. Spitzes Endstück nach einem der vorhergehenden Ansprüche, dadurch gekennzeichnet, daß die distale Öffnung (65) auf der gegenüberliegenden Seite des Endstückes zu der Rippe (80) liegt.

7. Spitzes Endstück nach Anspruch 6, dadurch gekennzeichnet, daß das Endstück ein Lumen (72) hat, welches sanft gekrümmt von der proximalen Öffnung zu dem distalen Ende der distalen Öffnung verläuft.

8. Spitzes Endstück nach Anspruch 7, umfassend einen Randflansch (58) zur Begrenzung der Einführung des Endstückes in einen Körpereinschnitt.

9. Spitzes Endstück nach Anspruch 8, gekennzeichnet durch einen zwischen den Enden liegenden Flansch.

10. Medizinische Schlauchleitung mit einem Schlauch (38) mit einem Endstück (54) nach einem der vorhergehenden Ansprüche, wobei der Schlauch mit dem proximalen Ende des Endstückes (54) verbunden ist.

11. Medizinische Schlauchleitung nach Anspruch 10, dadurch gekennzeichnet, daß das Endstück ein Lumen (72) hat, das glatt in ein Lumen (70) in dem Schlauch (38) übergeht.

12. Aorta-Kanüle (36) zur Einfürung in einen in eine Aorta eines Patienten gemachten Einschnitt zur Verbindung der Aorta mit einem außerkörperlichen Blutzirkulationssystem, mit einem Plastikschlauch (38), der ein Lumen (70) hat, das sich hierdurch erstreckt und ein proximales und ein distales Ende besitzt, Mitteln zur Verbindung des proximalen Endes (40) des Schlauches (38) mit dem außerkörperlichen Blutzirkulationssystems und einem im wesentlichen rohrförmigen Plastikendstück (54) mit einem proximalen Ende, das mit dem distalen Ende des genannten Schlauches verbunden ist, wobei das genannte Endstück (54) ein Lumen (72) hat, das mit dem genannten Schlauchlumen (70) und einer Öffnung in dessen Seitenwand für den Durchfluß von Blut verbunden ist, wobei das genannte Endstücklumen (72) eine das distale Ende hiervon abschließende Endwand hat, eine Rippe (80), die glatt abgerundete sich über die gesamte Länge erstreckende Kanten aufweist, die glatt in die äußere Oberfläche des distalen Endes des genannten Endstückes (54) übergehen, wobei das Endstück (54) aus relativ hartem Material derart gestaltet ist, daß das Material das genannte Endstück in einer festen Form während der Benutzung hält, dadurch gekennzeichnet, daß die distale Endwand sich von der Seitenwand des genannten Endstücklumens (72) zu dem distalen Ende der genannten Öffnung sanft krümmt, wobei die genannte Rippe (80) die einzige Rippe auf dem genannten Endstück ist und wobei das genannte Endstück ein Paar von sich längserstreckenden Ausnehmungen (82, 84) in desson Außenfläche aufweist, die an dessen distales Ende angrenzen und die im wesentlichen äußere axial sich erstreckende Rippe (80) zwischen den genannten Ausnehmungen (82, 84) zur Auseinanderbewegung der Aortawände am Einschnitt definieren, wobei die genannte Öffnung die einzige Öffnung in der Seitenwand des genannten Endstückes ist und die genannte Rippe (80) auf der Seite des genannten Endstückes angeordnet ist, die gegenüber der die genannte Öffnung einschließende Seite liegt, daß das genannte Endstück ein im wesentlichen zylindrisches Teil (62) in der Nähe des proximalen Endes der genannten Öffnung hat, wobei die radialen Außenflächen des genannten zylindrischen Teils (62) und der genannten Rippe (80) im wesentlichen radial äquidistant von der longitudinalen Achse des genannten Endstückes (54) verlaufen, daß das distale Ende des genannten Erdstückes (54) eine äußere sanft gekrümmte Oberfläche zwischen dem distalen Ende der genannten Öffnung und dem distalen Ende der genannten Rippe (80) hat und daß ein Flansch (58) vorgesehen ist, der mit dem genannten zylindrischen Teil (62) verbunden ist, welches sichradial auswärts hiervon erstreckt, und der in einem vorbestimmenten Abstand von der distalen End fläche des genannten Endstückes angeordnet ist, um die Einführung des genannten Endstückes (54) in die Aorta zu begrenzen , wobei die genannten Ausnehmungen einwärts konkav geformt sind.

13. Kanüle (36) nach Anspruch 12, dadurch gekennzeichnet, daß die genannten Ausnehmungen (82, 84) im wesentlichen elliptisch sind und sich mit ihren längeren Achsen über die gesamte Länge einwärts und zum distalen Ende des genannten Endstückes (54) erstrekken.

14. Kanüle (36) nach Anspruch 13, dadurch gekennzeichnet, daß die genannten Ausnehmungen (82, 84) im Querschnitt gekrümmt sind.

FIG. 1

EXTRACORPOREAL
CIRCULATION
SYSTEM

FIG. 3

56 60 65 8

58 64 62 8 82 80

FIG. 8

58 60 74 65

62 82 80 84

FIG. 4

56 62 65

58 60

FIG. 5

56 60 82

58 62 54 84 80

FIG. 6

65 58 60

82 80 84

42 56 58 60 54 64 74 80

70

38 36 66 72 62 65

FIG. 7

10

FIG. 2

FIG. 9

FIG. 10

FIG. 11